(19) Europäisches Patentamt
European Patent Office
Office européen des brevets

(11) Publication number: 0 502 447 A1

# (12) EUROPEAN PATENT APPLICATION

(21) Application number: 92103485.6

(22) Date of filing: 28.02.92

(51) Int. Cl.[5]: C07D 307/20, C07D 307/28, C07D 405/04, C07D 473/30, C07D 473/34

(30) Priority: 01.03.91 US 662686

(43) Date of publication of application: 09.09.92 Bulletin 92/37

(84) Designated Contracting States: AT BE CH DE DK ES FR GB GR IT LI LU MC NL PT SE

(71) Applicant: Bristol-Myers Squibb Company
345 Park Avenue
New York, N.Y. 10154(US)

(72) Inventor: Kim, Choung Un
13 High Field Lane
Madison, Connecticut 06443(US)
Inventor: Martin, John C.
116 Leslie Drive
San Carlos, California 94070(US)

(74) Representative: Kinzebach, Werner, Dr. et al
Patentanwälte Reitstötter, Kinzebach und Partner Sternwartstrasse 4 Postfach 86 06 49
W-8000 München 86(DE)

## (54) Tetrahydrofuran intermediates.

(57) Novel tetrahydrofuran and dihydrofuran derivatives useful as synthetic chemical intermediates and processes for their preparation.

EP 0 502 447 A1

## BACKGROUND OF THE INVENTION

This invention describes useful chemical intermediates and processes for their synthesis. These novel intermediate tetrahydro and dihydro furanyl compounds can be used in the manufacture of anti-cancer and anti-infective, and in particular, antiviral nucleoside compounds. One aspect of the usefulness of these intermediates concerns synthetic determination of the appropriate stereochemistry in the final nucleosidic products. Synthetic processes and chemical intermediates which can confer stereospecificity, or at least stereoselectivity, are of value in manufacturing nucleosides and nucleoside derivatives compared to processes requiring isomer separations.

The classical approaches to nucleoside derivative synthesis have employed variations comprising either the alteration of intact nucleosides or the attaching of a purine or pyrimidine base to a modified carbohydrate component. The useful furanyl intermediates of the present invention can be viewed as modified carbohydrate components whose chemical structure disposes them to facile conversion to stereochemically correct nucleosidic products.

Various furanyl-type synthetic intermediates have been described in the art.

Vial, et al., describes the use of thymine derivatives intermediates 1-3 in Nucleosides and Nucleotides, 9(2), 245-258(1990); as being incorporated

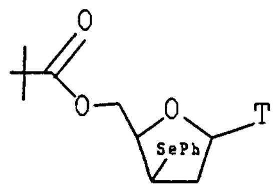
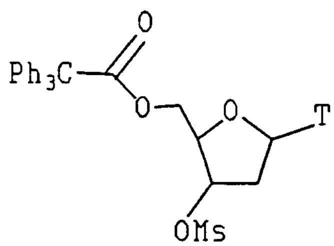
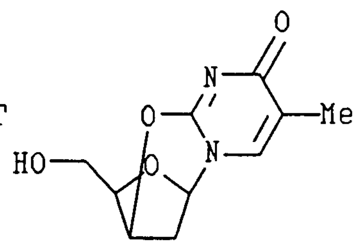

1 2 3

(T is the base thymine)

in processes to produce the anti-HIV agent 1-(2,3-dideoxy-β-D-glycero-pent-2-enofuranosyl)thymine (D4T).

Intermediates 4 and 5 are described by Wilson, et al,

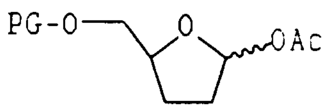
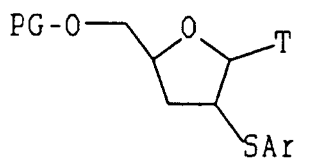

4 5

(PG is a protecting group; T is the base thymine)

in Tetrahedron Letters, 31/13, 1815-1818 (1990) as providing β-selectivity in processes for making D4T.

Intermediates 6 and 7 have been reported by Chu, et al,

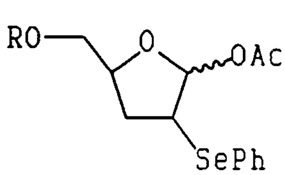
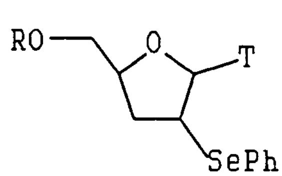

6 7

(R is a protecting group; T is the base thymine)

J. Org. Chem., 55/5, 1418-1420 (1990) in stereoselective processes for synthesizing 2',3'-dideoxy and 2',3'-dideoxy-2',3'-didehydronucleosides. Use of these previously described intermediates however require an isomeric separation step either prior to synthesis of the intermediate or in subsequent steps in the process directed to a nucleoside derivative.

Sato in Japanese Kokai JO 2069-469-A(06/09/88) disclosed dihydrofurans 8 and 9 and the procedures for their preparation.

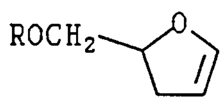
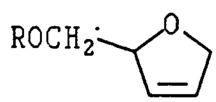

8 9

(R is benzyl or benzoyl)

These intermediates are disclosed as starting materials for making dideoxynucleosides.

The intermediates of the present invention are distinguishable over all these art compounds. The instant novel tetra and dihydrofurans provide advantages of improved stereochemical specificity for synthesizing desired nucleoside products as well as versatility, being applicable in the synthesis of various classes of nucleoside derivatives.

## Summary of The Invention

This invention comprises novel tetrahydrofuranyl and dihydrofuranyl compounds and processes for their preparation. These furanyl derivatives are useful chemical intermediates in chemical synthetic processes directed to nucleosidic analogs. These intermediates provide advantages in both end product versatility and selected stereochemical definition.

## Detailed Description of the Invention

The following flow chart, Scheme 1, illustrates the preparation of novel tetrahydrofuran derivatives of Formula I and II

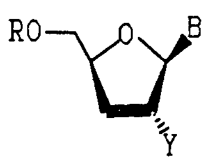
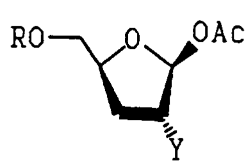

I II

and dihydrofuran derivatives of Formula III.

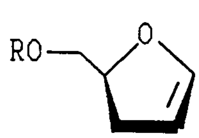

III

In Formulas I, II and III, R is a sterically bulky alkyl, acyl, silyl or arylalkyl group with pivaloyl, trityl, t-butyldiphenylsilyl and 4-monomethoxytrityl groups being preferred. In Formula I, Y is chloro, bromo or iodo; and B is either a pyrimidinyl group of Formula XI or a purinyl group of Formula XII.

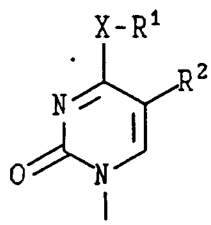

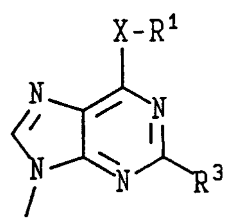

XI XII

Formula I compounds then may comprise either pyrimidine modifications of Formula Ia, or

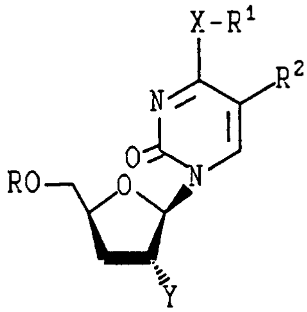

Ia

purinyl modifications of Formula Ib.

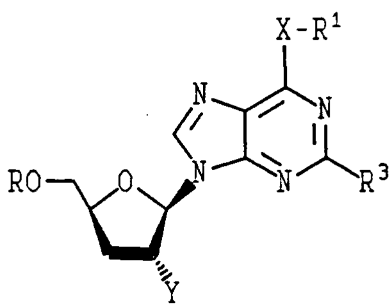

Ib

In structural Formulas Ia, Ib, XI, and XII; X refers to either -NH- or -O- linking moieties. $R^1$ can be either hydrogen or an organic synthetic protecting group of the type used to protect hydroxy and amino functional groups. For example, the primary alcohol or amino group on the pyrimidinyl or purinyl ring system may be protected as an ether or an ester when X is -O- or as an amide when X is -NH-. A preferred protection group is the trimethylsilyl group. The use of these and other organic synthetic protecting groups are well-known in the art and their use and chemistry is set forth in "Protective Groups in Organic Synthesis", T.W. Greene, John Wiley, New York, 1981.

The other substituent groups on the pyrimidinyl and purinyl rings are $R^2$ which can be hydrogen or methyl and $R^3$ which can be hydrogen or amino.

## Scheme 1

## Synthesis of Intermediates of Formula I

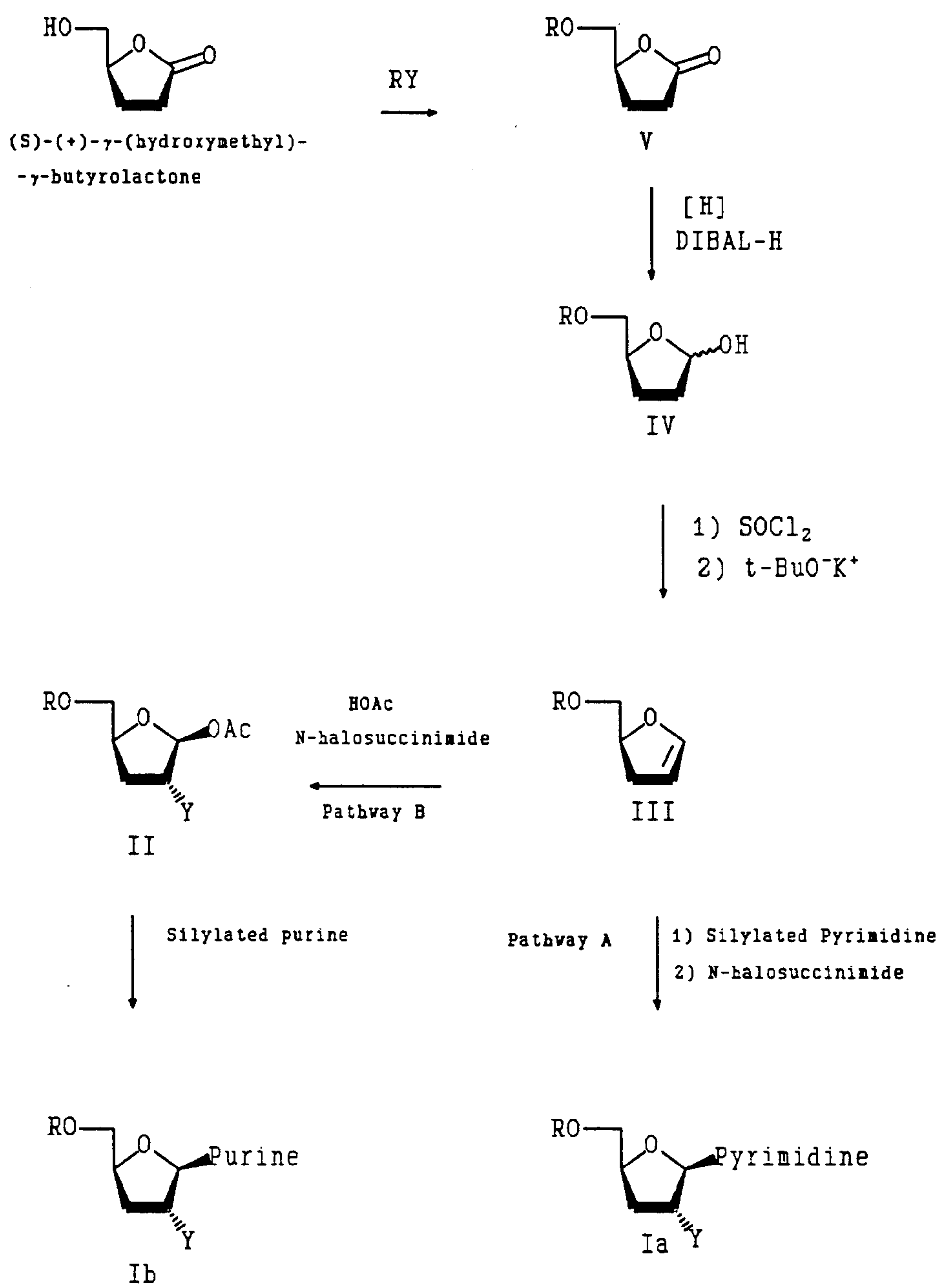


(S)-(+)-γ-(hydroxymethyl)-
-γ-butyrolactone

V

IV

III

II

Ib

Ia

In Scheme 1, the initial step involves protecting the pendant hydroxy group of the starting (S)-(+)-butyrolactone by silylation, acylation or alkylation with RY to give the lactone of Formula V. Many other methods of protecting a pendant hydroxyl group are known which also may be utilized. A preferred acylating agent is pivaloyl chloride. Lactone V is reduced with a selective hydride reducing agent such as diisobutylaluminum hydride (DIBAL-H) to give a mixture of α- and β- anomers of compound IV. The anomeric mixture is designated by use of the wavy line in structure IV. Dehydration of the lactol is effected by treatment with a halogenating agent such as thionyl chloride, followed by a strong base such as potassium t-butoxide or reaction of IV with oxalyl chloride followed by treatment with triethylamine gives the dihydrofuran intermediate III. Depending on whether a purinyl (Ib) or pyrimidinyl (Ia) product of Formula I is

desired; compound III is either reacted with a silylated pyrimidine, suitably protected, in the presence of an N-halosuccinimide such as N-iodosuccinimide (Pathway A) or is converted initially to the acetate intermediate II and then to the purinyl compound Ib (Pathway B).

The final steps shown in the process involve the use of either an activated pyrimidine or activated purine compound. These heterocyclic systems are activated synthetically by well-known methods in nucleoside chemistry which result in providing a silylated, acetylated or benzoylated base. In general, the heterocyclic base is activated by reaction of the pendant amino and hydroxy functionalities on the nucleus of the given base with silylating, acetylating, or benzoylating agents in standard methods familiar to one skilled in the art. Silylation is a preferred activating method. The $R^1$ group in purine bases is usually an acyl group, with benzoyl being preferred. The preferred activation of both purine and pyrimidine bases is achieved with silylation. Typical pyrimidine reagents used to provide Ia compounds comprise bis-2,4-trimethylsilylcytosine, bis-2,4-trimethylsilylthymine, and bis-2,4-trimethylsilyluracil. Some typical purine reagents which are reacted with the acetate compound II to provide Ib compound comprise 6-benzoylamino-9-trimethylsilylpurine, bis-6,9-trimethylsilylinosine, and tris-2,6,9-trimethylsilylguanine.

In summary, the synthetic process depicted in Scheme 1 comprises the following steps.

a) acylating (S)-(+)-γ-(hydroxymethyl)-γ-butyrolactone with RY to give a compound of Formula V;

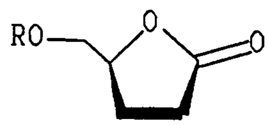

V

b) reducing compound V with diisobutylaluminum hydride or another selective hydride reducing agent to give an anomeric mixture of a compound of Formula IV;

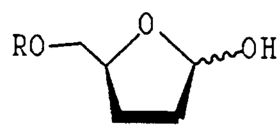

IV

c) converting the lactol IV to the corresponding dihydrofuranyl derivative III by treatment with thionyl chloride followed by potassium t-butoxide or with oxalyl chloride followed by triethylamine;

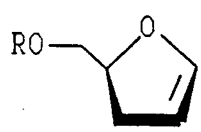

III

and

d) either following Pathway A and reacting compound III with a silylated pyrimidine derivative selected from bis-2,4-(trimethylsilyl)cytosine, bis-2,4-(trimethylsilyl)thymine, and bis-2,4-(trimethylsilyl)uracil; followed by successive treatment with an N-halosuccinimide such as N-iodosuccinimide and then aqueous bicarbonate solution to produce compound Ia

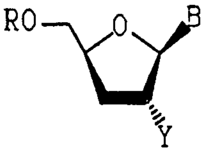

Ia

(B is a pyrimidinyl group of Formula XI as described supra); or following Pathway B and reacting compound III with acetic acid and then an N-halosuccinimide such as N-iodosuccinimide to give the acetate compound of Formula II.

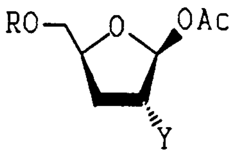

II

which is treated with a silylated purine derivative selected from 6-benzoylamino-9-trimethylsilylpurine, bis-6,9-trimethylsilylinosine, and tris-2,6,9-trimethylsilylguanine to produce compound Ib

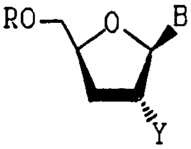

Ib

(B is a purinyl group of Formula XII as described supra).

## Description of Specific Embodiments

The process of this invention whereby the novel furanyl intermediates are prepared is illustrated in greater detail by the following examples directed to preferred embodiments of the hereinabove described process steps. These examples, however, should not be construed as limiting the scope of the present invention in any way.

### Example 1

(S)-γ-Pivaloyloxymethyl-γ-butyrolactone (V)

To a solution of (S)-γ-hydroxymethyl-γ-butyrolactone[1](40 g, 0.34M) in pyridine (200 mL) was added pivaloyl chloride (46 g, 0.38M) and the mixture was heated at 50°C for 5 h under nitrogen. The reaction was cooled to room temperature and MeOH (50 mL) was added. The mixture was then concentrated in vacuo, taken up in $CH_2Cl_2$-water. The $CH_2Cl_2$ was washed with water, 30% $H_3PO_4$, brine, and dried over $MgSO_4$. After removal of the solvent under reduced pressure, the residual oil was chromatographed on silica gel using $CH_2Cl_2$ as eluent to give 2 (48 g, 70%) as a colorless oil: $^1$H NMR ($CDCl_3$) δ 1.15 (s, 9H), 1.9-2.6 (m, 4H), 4.09 (dd, J = 4.8, 12.3 Hz, 1H), 2.29 (dd, J = 3.3, 12.3 Hz, 1H), 4.7-4.75 (m, 1H).

### Example 2

[1] Prepared according to the literature procedure: M. Taninguchi, K. Koga, S. Yamada, Tetrahedron, 30, 3547 (1974).

2-(R,S)-Hydroxy-5-(S)(pivaloyloxoymethyl)tetrahydrofuran (IV)

To a solution of (S)-γ-pivaloyloxymethyl-γ-butyrolactone (44.5 g, 0.22M) in ether (1L) and THF (150 mL) at -70 °C, under nitrogen, was added 1M diisobutylaluminum hydride (DIBAL-H) in THF (250 mL, 0.25M) over 90 min. After stirring at -70 °C for 2 h, MeOH (80 mL) was added and the reaction was warmed to room temperature and a 60% solution of potassium sodium tartrate (800 mL) was added. The mixture was stirred for 2 h, and the organic layer was separated, dried over $MgSO_4$, and concentrated in vacuo. The residual oil was chromatographed on silica gel using $CH_2Cl_2$-5% MeOH as eluent to give IV (37 g, 82%) as a colorless oil: $^1$H NMR ($CDCl_3$) δ 1.15 and 1.17 (s, 9H), 1.6-2.2 (m, 4H), 2.78 and 2.84 (broad s, 1H), 3.9-4.4 (m, 3H), 5.47 (d, J = 1.9 Hz, 0.5H), 5.55 (d, J = 3.9 Hz, 0.5H).

Example 3

(S)-5-Pivaloyloxymethyl-4,5-dihydrotetrahydrofuran (III)

To a solution of the lactol IV (5 g, 24.7 mmol) in $CH_2Cl_2$ (75 mL) was added thionyl chloride (6 g, 50 mmol) under nitrogen. After stirring at 23 °C for 2 h, volatiles were removed in vacuo. The oily residue was dissolved in toluene and evaporated to dryness to give the chloro compound as a colorless oil. This material was used for the next step without further purification.

To a solution of the chloro compound obtained from the lactol IV (5 g, 24.5 mmol) by the procedure described above, in THF (60 mL) at -70 °C was added potassium tert-butoxide (2.9 g, 25 mmol) portionwise under nitrogen. After stirring at -70 °C for 1 h, the reaction was quenched by addition of acetic acid (5 mL) and the mixture was warmed to room temperature. The reaction solution was evaporated in vacuo and the residual oil was taken up in $CH_2Cl_2$, washed with aqueous $NaHCO_3$, dried over $MgSO_4$, and evaporated to dryness. The residual liquid was distilled to give III (2.7 g, 60%), bp 60-68 °/4 Torr, as a colorless oil: NMR ($CDCl_3$) δ 1.18 (s, 9H), 2.28-2.39 (m, 1H), 2.60-2.75 (m, 1H), 4.08 (dd, J = 6.0, 11.7 Hz, 1H), 4.16 (dd, J = 4.5, 11.7 Hz, 1H), 4.65-4.75 (m, 1H), 4.85 (dd, J = 2.4, 4.8 Hz, 1H), 6.25 (dd, J = 2.4, 4.8 Hz, 1H); $^{13}$C NMR (50.3 MHz, $CDCl_3$) δ 27.369, 31.662, 44.806, 66.146, 77.736, 78.756, 99.423, 145.772.

| | | |
|---|---|---|
| Anal. Calcd. for $C_{10}H_{16}O_3$: | C, 65.19; | H, 8.75. |
| Found: | C, 65.51; | H, 8.57. |

Example 4

5'-0-Pivaloyl-2'-iodo-2',3'-dideoxy-5-methyluridine (Ia)

To a suspension of thymine (1.5 g, 12 mmol) in hexamethyldisilazane (40 mL) was added chlorotrimethylsilane (0.2 mL) and the mixture was heated at reflux with exclusion of moisture for 15 h. The resulting clear solution was evaporated in vacuo. The residual oil was dissolved in xylene (20 mL) and concentrated to dryness in vacuo. The residual clear oil was dissolved in THF (40 mL) and dihydrofuran III (1.84 g, 10 mmol) was added. A solution of N-iodosuccinimide (2.25 g, 10 mmol) in THF (20 mL) was added at -15 °C dropwise and the resulting yellow solution was allowed to stir for 90 min at -15 °C. The reaction was quenched by addition of aqueous $NaHCO_3$, and THF was concentrated under reduced pressure. The residual oil was taken up in ether, washed with water, agueous sodium bisulfite, dried over $MgSO_4$, and concentrated in vacuo to give Ia (4.1 g, 94%) as a yellow oil: $^1$H NMR ($CDCl_3$) δ 1.18 (s, 9H), 1.87 (s, 3H), 2.2-2.4 (m, 2H), 4.25 (dd, J = 3.3, 12.0 Hz, 1H), 4.2-4.3 (m, 1H), 4.39 (dd, J = 4.5, 12.0 Hz, 1H), 6.18 (d, J = 3.9 Hz, 1H), 7.21 (s, 1H).

Example 5

(2S, 3R, 5S)-2-Acetoxy-3-iodo-5-pivaloyloxymethyltetrahydrofuran (II)

To a solution of the dihydrofuran III (710 mg. 3.85 mmol) in $CH_2Cl_2$ (10 mL) at -20 °C, under nitrogen was added acetic acid (1.2 g, 20 mmol) followed by N-iodosuccinimide (877 mg, 3.85 mmol). After stirring at -20 °C for 60 min., the reaction was diluted with ether (50 mL), washed with aqueous $NaHCO_3$, and dried over $MgSO_4$. Evaporation of the dried solvents gave the iodo acetate intermediate II as a slightly yellow oil:

$^1H$ NMR ($CDCl_3$) $\delta$ 1.16 (s, 9H), 1.99 (s, 3H), 2.2-2.35 (m, 2H), 4.17 (d, J=4.2$H_Z$, 2H), 4.25 (d, J-4.5 $H_Z$, 1H), 4.65 (m, 1H), 6.38 (s, 1H). This material was used for further chemical transformation without purification.

Example 6

(2R,3R,5S)-6-N-Benzoyl-9-(tetrahydro-2-iodo-5-pivaloyloxymethyl-2-furanyl)adenine (Ib)

To a suspension of 6-N-benzoyladenine (87 mg, 3.5 mmol) in hexamethyldisilazine (15 mL) was added chlorotrimethylsilane (0.3 mL) and ammonium sulfate (30 mg) and the mixture was heated at 135°C for 18 h under nitrogen. The resulting clear solution was evaporation in vacuo with exclusion of moisture. The residual oil was dissolved in xylene (20 ml) and concentrated to dryness in vacuo. This residual clear oil and the iodo acetate intermediate II obtained by the procedure described above was dissolved in 1,2-dichloroethane (10 mL). A solution of 1.0 M tintetrachloride in $CH_2Cl_2$ (3.2 mL) was added dropwise at 20°C and the resulting yellow solution was allowed to stir for 60 min at -20°C then for 2 h without the cooling bath. The reaction was quenched by the addition of aqueous $NaHCO_3$ and diluted with $CH_2Cl_2$.The mixture was filtered and the organic phase was separated, dried over $MgSO_4$, and concentrated to dryness. The residual oil was chromatographed on silica gel using $CH_2Cl_2$ -5% MeOH as eluent to given Ib (650 mg 43%) as a white foam: $^1H$ NMR ($CDCl_3$) $\delta$ 1.67 (s, 9H), 2.4-2.6 (m, 2H), 4.35-4.4 (m, 2H), 4.78 (m, 1H), 5.11 (m, 1H), 6.46 (d, J=3.0 $H_z$, 1H), 7.4-7.6 (m, 3H), 8.0 (d, J=3.0 $H_z$, 2H), 8.18 (s, 1H), 8.98 (s, 1H).

By appropriate modification of reagents and using the experimental procedures set forth above, additional Formula I intermediates can be prepared.

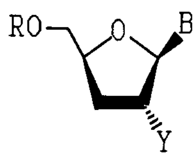

I

| Example No. | R | B | Y |
|---|---|---|---|
| 7. | pivaloyl | 1-cytosinyl | Br |
| 8. | trityl | 9-guaninyl | Cl |
| 9. | 4-monomethoxyltrityl | 9-adeninyl | Br |
| 10. | pivaloyl | 5-chloro-uracil | I |
| 11. | t-butyldiphenylsilyl | hypoxanthine | Br |
| 12. | pivaloyl | 5-ethyl-uracil | I |
| 13. | trityl | 2-amino-purinyl | Br |
| 14. | t-butyldimethylsilyl | 2,6-diaminopurinyl | I |

Thus, there is provided processes, as described above, for preparing novel tetrahydro- and dihydrofuranyl compounds.

**Claims**

**1.** A process for preparation of a tetrahydrofuran derivative of Formula I

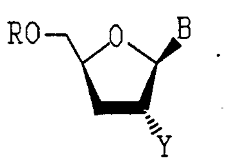

I

wherein R is a group selected from pivaloyl, t-butyldiphenylsilyl, trityl, and 4-monomethoxytrityl;

Y is selected from chloro, bromo and iodo; and

B is acetoxy, a pyrimidinyl group of Formula XI or a purinyl group of Formula XII,

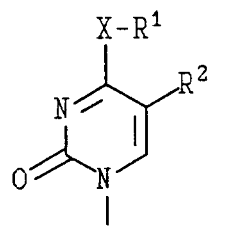

XI

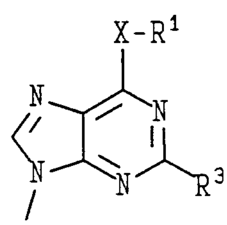

XII

in which X is -NH- or -O-;
$R^1$ is hydrogen or a protecting group;
$R^2$ is hydrogen or methyl; and
$R^3$ is hydrogen or amino;
the process comprising the steps of

a) acylating (S)-(+)-γ-(hydroxymethyl)-γ-butyrolactone with RX to give a compound of Formula V;

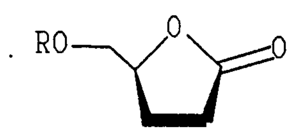

V

b) reducing compound V with a selective hydride reducing agent to give an anomeric mixture of a compound of Formula IV;

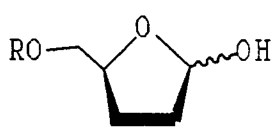

IV

c) reacting compound IV with thionyl chloride or oxalyl chloride followed by treatment with potassium t-butoxide or triethylamine to yield a 2,3-dihydrofuran compound of Formula III;

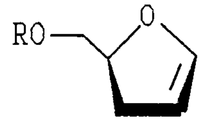

III

and

d)

(1) reacting compound III with a silylated pyrimidine derivative selected from bis-2,4-(trimethylsilyl)cytosine, bis-2,4-(trimethylsilyl)thymine, and bis-2,4-(trimethylsilyl)uracil; followed by successive treatment with an N-halosuccinimide and then aqueous bicarbonate solution to produce compound Ia;

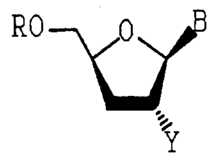

Ia

or

(2) reacting compound III with acetic acid and then an N-halosuccinimide to give the acetate compound of Formula II.

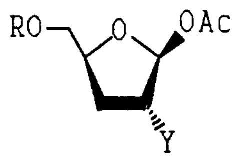

II

which is treated with a silylated purine derivative selected from 6-benzoylamino-9-trimethylsilylpurine, bis-6,9-trimethylsilylinosine, and tris-2,6,9-trimethylsilylguanine to produce compound Ib

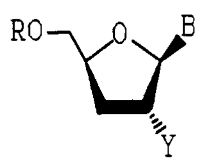

Ib

**2.** The process of claim 1 wherein B is a pyrimidinyl group.

**3.** The process of claim 1 wherein B is a purinyl group.

**4.** The process of claim 1 wherein R is pivaloyl.

**5.** The compound of Formula III

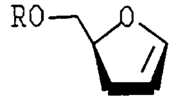

III

wherein R is a group selected from t-butyl, pivaloyl, t-butyldiphenylsilyl, trityl and 4-monomethoxytrityl.

**6.** The compound of Formula II

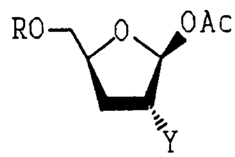

II

wherein Ac is acetyl; Y is chloro, bromo and iodo; and R is a group selected from pivaloyl, t-butyldiphenylsilyl, trityl and 4-monomethoxytrityl.

**7.** The compound of Formula I

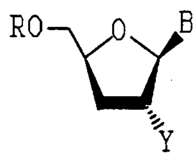

I

wherein Y is chloro, bromo and iodo; R is a group selected from pivaloyl, t-butyldiphenylsilyl, trityl and 4-monomethoxytrityl; and B is a pyrimidinyl group of Formula XI or a purinyl group of Formula XII,

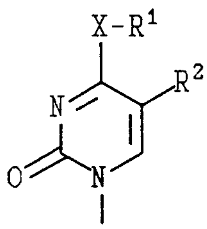

XI

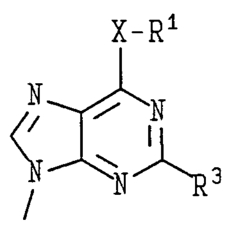

XII

in which X is -NH- or -O-;
$R^1$ is hydrogen or a protecting group such as trimethylsilyl and benzoyl;
$R^2$ is hydrogen or methyl; and
$R^3$ is hydrogen or amino.

European Patent Office

# EUROPEAN SEARCH REPORT

Application Number

EP 92 10 3485
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 398 230 (BRISTOL-MYERS SQUIBB COMPANY)<br>* page 3 - page 4, line 50 *<br>--- | 7 | C07D307/20<br>C07D307/28<br>C07D405/04<br>C07D473/30<br>C07D473/34 |
| A | EP-A-0 355 031 (AKADEMIE DER WISSENSCHAFTEN DER DDR)<br>* page 3 - page 5 *<br>--- | 7 | |
| X | EP-A-0 311 694 (RIKAGAKU KENKYUSHO)<br>* page 4 - page 5, line 12 *<br>* page 11; example A10 *<br>--- | 7 | |
| A,D | PATENT ABSTRACTS OF JAPAN<br>vol. 14, no. 250 (C-723)(4193) 29 May 1990<br>& JP-A-2 069 476 ( KOHJIN CO LTD ) 8 March 1990<br>* abstract *<br>--- | 5,7 | |
| A | PATENT ABSTRACTS OF JAPAN<br>vol. 14, no. 543 (C-783)(4486) 30 November 1990<br>& JP-A-2 229 192 ( KOHJIN CO LTD ) 11 September 1990<br>* abstract *<br>--- | 5,7 | TECHNICAL FIELDS SEARCHED (Int. Cl.5 ) |
| A,D | TETRAHEDRON LETTERS<br>vol. 31, no. 13, 1990, GREAT BRITAIN<br>pages 1815 - 1818;<br>LAWRENCE J.WILSON ET AL.: 'A general method for controlling Glycosylation Stereochemistry in the synthesis of 2'-deoxyribose nucleosides'<br>* the whole document *<br>--- | 1,6,7 | C07D |
| X,D | THE JOURNAL OF ORGANIC CHEMISTRY<br>vol. 55, no. 5, 2 March 1990, COLUMBUS OHIO<br>pages 1418 - 1419;<br>CHUNG K.CHU ET AL.: 'A highly Stereoselective Glycosylation of 2-(phenylselenenyl)-2,3-dideoxyribose Derivative with Thymine:Synthesis of 3'-deoxy-2',3'-didehydrothymidine and 3'-deoxythymidine'<br>* the whole document * | 1 | |

The present search report has been drawn up for all claims

| Place of search | Date of completion of the search | Examiner |
|---|---|---|
| BERLIN | 02 JUNE 1992 | KYRIAKAKOU G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 92 10 3485
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | --- | | |
| X | TETRAHEDRON<br>vol. 46, 1990, GREAT BRITAIN<br>pages 4503 - 4516;<br>ANDREW TAKLE ET AL.: 'A new approach to 1,7-dioxaspiro(5,5)undec-4-enes via metallated allenol ethers.Synthesis of Lacrimin A.'<br>Scheme 4<br>* page 4505; examples 19,20 * | 1,5 | |
| | ----- | | |
| | | | TECHNICAL FIELDS SEARCHED (Int. Cl.5) |

The present search report has been drawn up for all claims

| Place of search | Date of completion of the search | Examiner |
|---|---|---|
| BERLIN | 02 JUNE 1992 | KYRIAKAKOU G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)